# EUROPEAN PATENT SPECIFICATION

(11) **EP 2 205 188 B1**
(45) Date of publication and mention of the grant of the patent: **09.04.2014**
(21) Application number: 08833682.1
(22) Date of filing: 24.09.2008
(51) Int. Cl.: A61F 2/38

(54) **CEMENTLESS TIBIAL TRAY**
ZEMENTFREIES TIBIAPLATEAU
PLATEAU TIBIAL SANS CIMENT

(30) Priority: 25.09.2007 US 975012 P
(43) Date of publication of application: 14.07.2010
(73) Proprietor: Biomet Manufacturing Corp., Warsaw, IN 46582 (US)
(72) Inventor: UTHGENANNT, Brian A., Winona Lake Indiana 46590 (US); METZGER, Robert, Wakarusa Indiana 46573 (US); HERSHBERGER, Troy W., Winona Lake Indiana 46590 (US)
(74) Representative: Robin, Gemma Claire
(86) International application number: PCT/US2008/011071
(87) International publication number: WO 2009/042150

(56) References cited:
- EP-A- 1 065 034
- EP-A- 1 421 918
- US-A1- 2006 224 244
- US-A1- 2006 235 517

## Description

This application is related to U.S. Provisional Application No. 60/975,012, filed on September 25, 2007.

### FIELD

The present teachings relate to a method of making a tibial tray.

### BACKGROUND

Porous metal implants or implants having porous metal portions are used to promote ingrowth of surrounding bony tissue and soft tissues into the implant. When the porosity, integrity and continuity of the metals are sufficient, porous implants serve as a scaffold for tissue ingrowth to provide the desired fixation to host bone. The porous material can be formed by removing pieces from a metal substrate, such as by etching a solid piece of metal. The porous material can also be formed by using small metal particles such as powders. A method of making a tibial tray, comprising forming a substrate having a superior surface, attaching a polymer portion onto a porous metal material and attaching said material onto said surface is known from the document US 2006/224244 A1.

The present teachings provide a method for making a tibial tray. According to one example, a substrate having a superior surface can be formed. Porous metal material can be attached onto the superior surface of the substrate. Selected areas of the substrate can be removed to form first features of the tibial tray. Selected areas of the polymer portion can be removed to form second features of the tibial tray.

According to additional features, attaching the porous metal material can include sintering the porous metal material onto the superior surface of the substrate. Attaching the polymer portion can include molding the polymer portion onto the porous metal material. Removing selected areas of the substrate can include forming portions of a tibial stem. Removing selected areas of the polymer portion can comprise forming a superior surface of the tibial tray including attachment features adapted for selectively securing a bearing.

According to other features, a foil barrier can be attached intermediate the porous metal portion and the polymer portion.

Further areas of applicability of the present teachings will become apparent from the detailed description provided hereinafter. It should be understood that the detailed description and specific examples, are intended for purposes of illustration only and are not intended to limit the scope of the teachings.

### BRIEF DESCRIPTION OF THE DRAWINGS

FIG. 1 is an anterior view of a substrate according to the present teachings;
FIG. 2 is an anterior view of the substrate of FIG. 1 shown with porous metal material attached thereto;
FIG. 3A is an anterior view of the substrate and porous metal material of FIG. 2 and shown with a polymer portion attached thereto;
FIG. 3B is an anterior view of the substrate and porous metal material of FIG. 2 and shown with a polymer portion attached to a foil barrier extending along the porous metal material according to additional features;
FIG. 4 is an anterior view of the substrate, porous metal and polymer member of FIG. 3A and shown with a portion of the substrate removed;
FIG. 5 is an anterior view of a tibial tray constructed in accordance with the present teachings, the tibial tray shown with selected portions of the polymer portion and substrate portion removed to create additional features of the tibial tray;
FIG. 6 is an anterior view of an exemplary knee joint prosthesis including the tibial tray of FIG. 5; and
FIG. 7 illustrates an exemplary sequence of manufacturing the tibial tray of FIG. 5.

### DETAILED DESCRIPTION

The following description is merely exemplary in nature and is in no way intended to limit the teachings, their application, or uses. Although various embodiments may be described in conjunction with a porous metal implant for use with a tibial tray, it is understood that the implants and methods of the teachings can be of any appropriate substrate or shape and can be used with any appropriate procedure and not solely those illustrated.

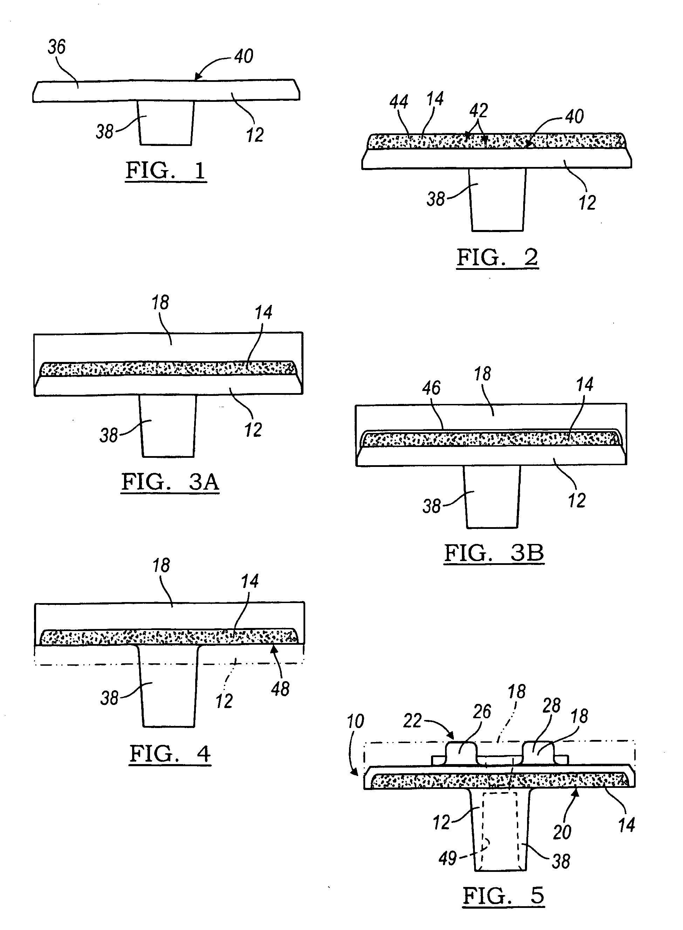

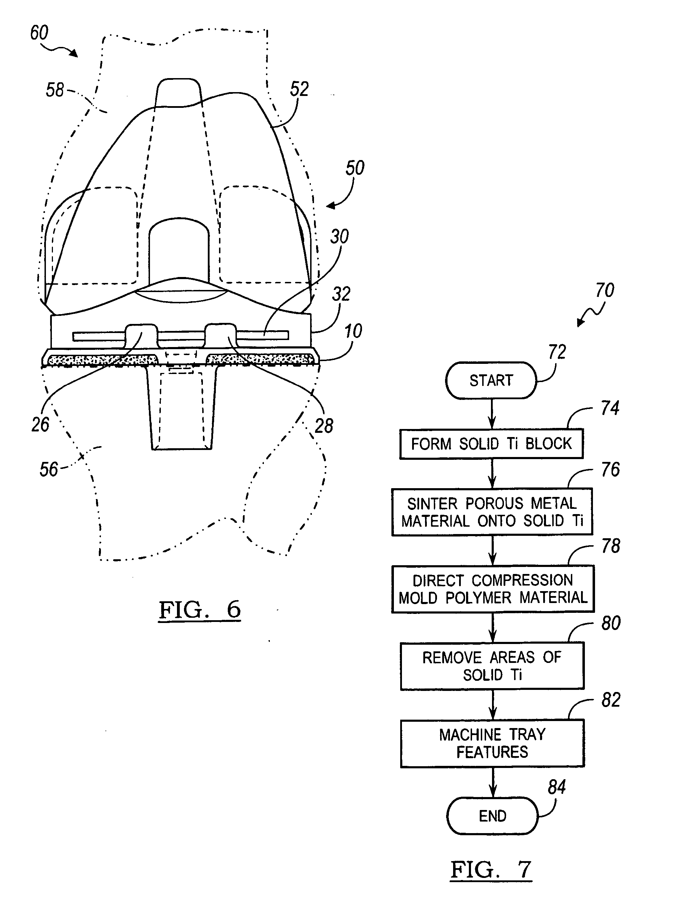

Referring initially to FIG. 5, a tibial tray constructed in accordance with the present teachings is shown and generally identified at reference numeral 10. The tibial tray 10 can generally include a solid metal substrate portion 12, a porous metal portion 14, and a polymer portion 18. As will be described, the tibial tray 10 can be one-piece and formed from a sequential manufacturing process. The tibial tray 10 can provide a porous metal inferior surface 20 conducive to bony ingrowth. The polymer portion 18 can define a superior portion 22 of the tibial tray 10. The tibial tray 10 can include a pair of integrally formed posts 26 and 28, formed by the polymer portion 18, and which extend superiorly at an anterior edge of the tibial tray 10. The posts 26 and 28 can define grooves (not specifically shown) operable to receive a locking bar 30 (FIG. 6) which are able to secure a tibial insert 32 (FIG. 6) to the tibial tray 10 in a manner described below.

With reference now to FIGS. 1-5, an exemplary method of forming the tibial tray will be described. At the outset, the substrate 12 may be formed having a generally planar upper portion 36 and a cylindrical post portion 38. The substrate 12 can be formed of solid biocompatible material such as, but not limited to titanium. The substrate 12 can be formed into the shape shown in FIG. 1 by any suitable means such as by machining, molding, casting or other methods. As used herein, the term "molding" is used to refer to any molding process, such as, but not limited to, injection molding or (direct) compression molding.

Referring to FIG. 2, the porous metal material 14 can be attached to a superior surface 40 of the upper portion 36. In one example, the porous metal material 14 can be formed from a mixture of a metal powder, a spacing agent (not shown), and a non-polar liquid binder (not shown). The porous metal material 14 can be formed by heating the mixture to a temperature sufficient to remove the spacing agent and non-polar liquid binder thereby leaving a plurality of pores 42 between the interconnected metal powder particles 44.

The porous metal material 14 can be any metal or alloy that is suitable for use as an implant and provides the desired strength, load bearing capabilities, and ability to become porous. Suitable exemplary metals include titanium, cobalt, chromium, or tantalum, alloys thereof, stainless steel, and combinations thereof. The metal powder particles 44 can have a diameter of from about 5 micrometers to about 1500 micrometers. In various embodiments, the metal powder 44 can be of at least two different particle sizes.

The spacing agent can occupy space that gives rise to the pores 42 of the porous metal material 14. The spacing agent can be removable from the mixture and it may be desirable if the spacing agent does not leave residue in the porous metal material 14. It may be further desirable that the spacing agent expands or contracts to supplement the formation of pores 42 of a desired size within the porous metal material 14. The spacing agent can be selected from the group consisting of hydrogen peroxide, urea, ammonium bicarbonate, ammonium carbonate, ammonium carbamate, calcium hydrogen phosphate, naphthalene, and mixtures thereof, or can be any other suitable subliming and space forming material. Generally, the spacing agent can have a melting point, boiling point, sublimation temperature, etc. of about less than 250°C. The spacing agent can provide the macroporosity and microporosity of the biocompatible metal powder before and during the thermal cycling processes, because after the spacing agent decomposes and metallurgical bonds form between the metal powder particles 44, pores (i.e., the pores 42) or gaps remain where the spacing agent was located. One suitable porous metal and method for making may be found in U.S. Pat. App. Serial No. 11/357,929, filed February 17, 2006, entitled "Method and Apparatus for Forming Porous Metal Implants" owned by Biomet Manufacturing Corp. of Warsaw, Indiana.

Altering the ratios of the mixture components and/or the sizes of the components can provide a porous metal material 14 having a higher or lower porosity, enhanced load-bearing abilities, optimal bone ingrowth abilities and can help to tailor the porous metal material 14 for a particular region of the body (such as a knee according to the instant example). Utilizing a ratio of metal powder to a spacing agent of 8:1 can provide a relatively dense porous metal material 14 having very fine pores. In another example, in a mixture having a 3:1 metal powder to spacing agent ratio, if the spacing agent has a diameter of at least about 25 micrometers and the metal powder has a diameter of about 10 micrometers, large pores result. If the metal powder and spacing agent diameter sizes were reversed, smaller pores would result. It is appreciated that such configurations are merely exemplary and the porous metal material 14 can have any suitable porosity. For example, in embodiments, the porous metal material 14 can include pores ranging in size from about 100 microns to about 600 microns. In embodiments, the size of the pores can average about 300 microns.

The mixture can also include metal powders of different particulate sizes. By including metal powder particulates of at least two different sizes, a porosity gradient can be achieved. The porosity gradient can be such that the porosity of the porous metal material 14 increases or decreases by up to about 80% across the thickness of the porous metal material 14. The porosity gradient can be continuous and scale up (or down) to a desired amount, or the porosity gradient can include differing porosity regions (e.g., 80% porosity region transitions to a 40% porosity region which transitions to a 75% porosity region). The transitions between the regions can be continuous in the porous metal material 14. To provide the different porosities, a mixture corresponding to a particular porosity can be stacked on top of or adjacent to a mixture having a different porosity.

The porous metal material 14 can be attached to the substrate 12 by any suitable means, such as welding, sintering, using a laser, etc. In various embodiments, the substrate 12 can be formed of metal such as the same metal as the porous metal material 14. The temperature and pressure conditions used to attach the porous metal material 14 to the substrate 12 can be such that diffusion and metallurgical bonding between the substrate surface areas and the adjacent porous metal surfaces will be achieved. For example, in an embodiment where the porous metal portion 14 and the metal substrate 12 are heated to 1000°C, the pressure applied must be such that the resultant structure has structural integrity for implanting into a recipient without significant defects.

The substrate 12 can be prepared prior to attaching the porous metal material 14. The substrate 12 can be acid etched, subjected to an acid bath, grit blasted, or ultrasonically cleaned for example. Other preparations include adding channels, pits, grooves, indentations, bridges, or holes to the substrate 12. These additional features may increase the attachment of the porous metal material 14 to the underlying substrate 12.

Additional agents can be coated onto or in at least a surface of the porous metal material 14. Agents include resorbable ceramics, resorbable polymers, antibiotics, demineralized bone matrix, blood products, platelet concentrate, allograft, xenograft, autologous and allogeneic differentiated cells or stem cells, nutrients, peptides and/or proteins, vitamins, growth factors, and mixtures thereof, which would facilitate ingrowth of new tissue into the porous metal material 14. For example, if the additional agent is a peptide, an RGB peptide can be advantageously incorporated into the porous metal material 14.

Turning now to FIG. 3A, the polymer portion 18 may be attached to the porous metal material 14. In one example, the polymer portion 18 may be molded into the porous metal material 14. According to one example, the polymer portion 18 can include polyetheretherketone (PEEK) and/or carbon fiber reinforced PEEK (CFR-PEEK). According to additional features, a foil barrier 46 (FIG. 3B) may be provided intermediate the polymer portion 18 and the porous metal material 14. The foil barrier 46 can be separately formed and disposed onto the porous metal material 14. In another example, the upper superior surface of the porous metal material 14 can be smoothed out or "smeared", such as by a machining operation to remove or substantially remove any porosity from the superior surface of the porous metal material 14. In another example, the polymer portion 18 can be molded or machined separately and subsequently attached to the superior surface of the porous metal material 14 or to the foil barrier 46. Further, in embodiments, the foil barrier 46 may be located within the porous metal material 14, thereby allowing the polymer portion 18 to mold into the porous metal material 14 while also preventing the polymer portion 18 from molding completely through the porous metal material 14.

Next, with reference to FIG. 4, a portion of the substrate 12 substantially corresponding to the upper portion 36 is removed. In one example, the substrate 12 may be machined away revealing the porous metal portion 14 on an inferior surface 48. With reference now to FIG. 5, the final features of the tibial tray 10 are formed. In one example, an area of PEEK 18 is removed (such as machined away, etc.) to form the posts 26 and 28. Likewise, a female taper 49 may be formed on the post portion 38. Other features not specifically shown may be machined, lasered or otherwise created on the tibial tray 10. In one alternate example, the substrate 12 including the polymer portion 18 and the post portion 38 (and optionally the final features such as the posts 26 and 28, etc.) can be initially formed. Next, the porous metal material 14 can be separately formed. The porous metal material 14 can subsequently be coupled to the substrate by any suitable method. In one example, the porous metal material 14 can have an aperture formed centrally therethrough for receiving the post portion 38 during an assembly step. In one implementation, the aperture can be tapered for cooperatively mating with the outer tapered geometry of the post portion 38 such as in a Morse taper connection.

With reference to FIG. 6, the tibial tray 10 is shown in an implanted position as part of a knee joint prosthesis 50. The knee joint prosthesis 50 can include the tibial tray 10, the bearing 32, and a femoral component 52. The knee joint prosthesis 50 is functionally depicted as being secured to a tibia 56 and a femur 58 of a surgically resected knee joint 60. In one example, the tibial tray 10 can be cementless. The post 38 of the tibial tray 10 can be inserted into an opening made by the surgeon in the longitudinal center of the tibia 56. The bearing 32 can define complementary recesses (not specifically shown) adapted to receive the posts 26 and 28 of the tibial tray 10. Another recess (not specifically shown) may be formed on the bearing 32 for aligning with the grooves on the first and second posts 26 and 28 so as to receive the locking bar 30. It is appreciated that the tibial tray 10 can be formed and/or adapted for use with any kind of knee replacement such as a posterior stabilized (PS) knee, crutiate retaining (CR) knee, hinged knee, fixed knee and others.

An exemplary method of forming the tibial tray 10 is referred to generally at reference 70 in a flow diagram shown in FIG. 7. The method 70 begins in step 72. In step 74, the substrate 12 is formed such as from a solid block of titanium. In step 76, the porous metal material 14 is sintered onto the substrate 12. In step 78, the polymer material 18 can be molded onto the porous metal material 14. In step 80, areas of the substrate 12 can be removed. In step 82, features of the tray 10 can be formed into the polymer material 18 and/or the porous metal material 14. The method ends in step 84.

According to an additional method which is not in accordance with the present invention, the porous metal material 14 may be sintered into a shape without requiring a substrate (i.e. such as substrate 12). In one example, the cylindrical post portion 38 can be formed of porous metal material 14. Next, the polymer material 18 can be molded onto the porous metal material 14. The features of the tray can then be machined into the polymer material 18 and/or the porous metal material 14. In another method of forming the tibial tray 10, the substrate 12 can be molded or machined separately from the porous metal material 14. The two pieces can then be joined by way of a thermal expansion process followed by a cooling retraction process. In one example, at least one of the substrate 12 and the porous metal material 14 can be heated. The substrate 12 can then be placed onto the porous metal material 14 and at least one of the substrate 12 and the porous metal material 14 are cooled (and/or left to return to an ambient temperature). Interlocking features (not specifically shown) formed on the substrate 12 and/or the porous metal material 14 can initially expand (from the heating process) and subsequently contract (from cooling) ultimately interlocking the substrate 12 and the porous metal material 14. One method of such a process is discussed in detail in commonly owned U.S. Patent Application No. 12/038,570, filed February 27, 2008.

Those skilled in the art can now appreciate from the foregoing description that the broad teachings of the present disclosure can be implemented in a variety of forms. Therefore, while this disclosure has been described in connection with particular examples thereof, the true scope of the disclosure should not be so limited since other modifications will become apparent to the skilled practitioner upon a study of the drawings, the specification and the following claims.

## Claims

1. A method of making a tibial tray, the method comprising:
forming a substrate (12) having a superior surface;
attaching porous metal material (14) onto the superior surface;
attaching a polymer portion (18) onto the porous metal material; and
removing selected areas of the substrate to form first features of the tibial tray; or removing selected areas of the substrate to form first features of the tibial tray
and selected areas of the polymer portion to form second features of the tibial tray.

2. The method of claim 1 wherein the removing comprises removing both of the selected areas of the substrate to form first features of the tibial tray and the selected areas of the polymer portion to form second features of the tibial tray

3. The method of claim 1 wherein attaching the porous metal material comprises sintering the porous metal material onto the superior surface of the substrate.

4. The method of claim 1 wherein attaching the polymer portion comprises molding the polymer portion onto the porous metal material.

5. The method of claim 2 wherein removing selected areas of the substrate comprises forming portions of a tibial stem.

6. The method of claim 2 wherein removing selected areas of the substrate comprises exposing at least portions of the porous metal material.

7. The method of claim 1 wherein removing selected areas of the polymer portion comprises forming a superior surface of the tibial tray including attachment features adapted for selectively securing a bearing.

8. The method of claim 1, further comprising attaching a foil barrier (46) intermediate the porous metal material (14) and the polymer portion (18).

9. The method of claim 8 wherein attaching the foil barrier comprises attaching the foil barrier onto the porous metal material.

10. The method of claim 1 wherein attaching the polymer portion comprises heating at least one of the polymer portion and the porous metal material, placing the polymer portion onto the porous metal material, and cooling at least one of the polymer portion and the porous metal material.

11. The method of claim 1 wherein forming the substrate comprises:
forming a titanium substrate having a first superior surface and wherein the porous metal material is attached to the first superior surface by applying at least one of heat or pressure to at least one of the substrate or porous metal material, the porous metal material having a second superior surface.

12. The method of claim 11 wherein attaching the polymer portion comprises:
molding polyetheretherketone (PEEK) onto the second superior surface.

13. The method of claim 12 wherein removing selected areas of the polymer portion comprises;
removing selected areas of the PEEK to form attachment features that are configured to selectively secure a bearing and to expose at least one portion of the porous metal material.

14. The method of claim 11 wherein attaching the porous metal material comprises welding the porous metal material to the first superior surface of the substrate.

15. The method of claim 11 wherein attaching the porous metal material comprises one of diffusion or metallurgical bonding the substrate to the porous metal material.

## Patentansprüche

1. Verfahren zur Herstellung eines Tibiaplateaus, wobei das Verfahren umfasst:
Bilden eines Substrates (12) mit einer oberen Fläche;
Befestigen von porösem Metallmaterial (14) auf der oberen Fläche;
Befestigen eines Polymerteils (18) auf dem porösen Metallmaterial; und
Entfernen ausgewählter Bereiche des Substrates, so dass man erste Funktionen des Tibiaplateaus erhält; oder Entfernen ausgewählter Bereiche des Substrates, so dass man erste Funktionen des Tibiaplateaus erhält, und ausgewählter Bereiche des Polymerteils, so dass man zweite Funktionen des Tibiaplateaus erhält.

2. Verfahren nach Anspruch 1, wobei man beim Entfernen sowohl die ausgewählten Bereiche des Substrates entfernt, so dass man erste Funktionen des Tibiaplateaus erhält, als auch die ausgewählten Bereiche des Polymerteils, so dass man zweite Funktionen des Tibiaplateaus erhält.

3. Verfahren nach Anspruch 1, wobei man beim Befestigen des porösen Metallmaterials das poröse Metallmaterial auf die obere Fläche des Substrates sintert.

4. Verfahren nach Anspruch 1, wobei man beim Befestigen des Polymerteils den Polymerteil auf das poröse Metallmaterial formt.

5. Verfahren nach Anspruch 2, wobei man beim Entfernen ausgewählter Bereiche des Substrates Teile eines Tibiastamms erhält.

6. Verfahren nach Anspruch 2, wobei man beim Entfernen ausgewählter Bereiche des Substrates zumindest Teile des porösen Metallmaterials freilegt.

7. Verfahren nach Anspruch 1, wobei man beim Entfernen ausgewählter Bereiche des Polymerteils eine obere Fläche des Tibiaplateaus erhält, die Befestigungsfunktionen aufweist, die sich zum selektiven Sichern eines Lagers eignen.

8. Verfahren nach Anspruch 1, wobei man zwischen dem porösen Metallmaterial (14) und dem Polymerteil (18) zudem eine Sperrfolie (46) befestigt.

9. Verfahren nach Anspruch 8, wobei man beim Befestigen der Sperrfolie die Sperrfolie auf dem porösen Metallmaterial befestigt.

10. Verfahren nach Anspruch 1, wobei man beim Befestigen des Polymerteils mindestens einen Bestandteil von Polymerteil und porösem Metallmaterial erwärmt, den Polymerteil auf dem porösen Metallmaterial unterbringt, und mindestens einen Bestandteil von Polymerteil und porösem Metallmaterial kühlt.

11. Verfahren nach Anspruch 1, wobei das Bilden des Substrates umfasst:
Bilden eines Titansubstrates mit einer ersten oberen Fläche, wobei das poröse Metallmaterial an der ersten oberen Fläche befestigt wird, indem man mindestens eine Größe von Hitze oder Druck auf mindestens einen Bestandteil von Substrat oder porösem Metallmaterial ausübt, wobei das poröse Metallmaterial eine zweite obere Fläche hat.

12. Verfahren nach Anspruch 11, wobei das Befestigen des Polymerteils umfasst:
Formen von Polyetheretherketon (PEEK) auf der zweiten oberen Fläche.

13. Verfahren nach Anspruch 12, wobei das Entfernen ausgewählter Bereiche des Polymerteils umfasst:
Entfernen ausgewählter Bereiche des PEEK, so dass man Befestigungsfunktionen erhält, die so konfiguriert sind, dass sie selektiv ein Lager sichern und mindestens einen Teil des porösen Metallmaterials freilegen.

14. Verfahren nach Anspruch 11, wobei man beim Befestigen des porösen Metallmaterials das poröse Metallmaterial an die erste obere Fläche des Substrates schweißt.

15. Verfahren nach Anspruch 11, wobei man beim Befestigen des porösen Metallmaterials das Substrat durch Diffusions- oder metallurgisches Schweißen an das poröse Metallmaterial bindet.

## Revendications

1. Procédé de fabrication d'un plateau tibial, le procédé comprenant :
former un substrat (12) ayant une surface supérieure ;
fixer un matériau métallique poreux (14) à la surface supérieure ;
fixer une portion polymère (18) sur le matériau métallique poreux ; et
retirer des zones sélectionnées du substrat pour former des premières propriétés du plateau tibial ; ou retirer des zones sélectionnées du substrat pour former des premières propriétés du plateau tibial, et
des zones sélectionnées de la portion polymère pour former des deuxièmes propriétés du plateau tibial.

2. Procédé selon la revendication 1, dans lequel le retrait comprend le retrait à la fois des zones sélectionnées du substrat pour former des premières propriétés du plateau tibial et des zones sélectionnées de la portion de polymère pour former des secondes propriétés du plateau tibial.

3. Procédé selon la revendication 1, dans lequel la fixation du matériau métallique poreux comprend le frittage du matériau métallique poreux sur la surface supérieure du substrat.

4. Procédé selon la revendication 1, dans lequel la fixation de la portion polymère comprend le moulage de la portion polymère sur le matériau métallique poreux.

5. Procédé selon la revendication 2, dans lequel le retrait de zones sélectionnées du substrat comprend la formation de portions d'une tige tibiale.

6. Procédé selon la revendication 2, dans lequel le retrait de zones sélectionnées du substrat comprend l'exposition au moins de portions du matériau métallique poreux.

7. Procédé selon la revendication 1, dans lequel le retrait de zones sélectionnées de la portion polymère comprend la formation d'une surface supérieure du plateau tibial incluant les propriétés de fixation aptes à assurer sélectivement un support.

8. Procédé selon la revendication 1, comprenant en outre la fixation d'une barrière de feuille (46) entre le matériau métallique poreux (14) et la portion polymère (18).

9. Procédé selon la revendication 8, dans lequel la fixation de la barrière de feuille comprend la fixation de la barrière de feuille au matériau métallique poreux.

10. Procédé selon la revendication 1, dans lequel la fixation de la portion polymère comprend le chauffage au moins d'un parmi la portion polymère et le matériau métallique poreux, le placement de la portion polymère sur le matériau métallique poreux et le refroidissement d'au moins un parmi la portion polymère et le matériau métallique poreux.

11. Procédé selon la revendication 1, dans lequel la formation du substrat comprend :
former un substrat en titane ayant une première surface supérieure, et où le matériau métallique poreux est fixé à la première surface supérieure an appliquant au moins une parmi la chaleur et la pression à au moins un parmi le substrat ou le matériau métallique poreux, le matériau métallique poreux ayant une deuxième surface supérieure.

12. Procédé selon la revendication 11, dans lequel la fixation de la portion polymère comprend :
mouler du poly-éther-éther-cétone (PEEK) sur la deuxième surface supérieure.

13. Procédé selon la revendication 12, dans lequel le retrait de zones sélectionnées de la portion polymère comprend :
le retrait de zones sélectionnées du PEEK pour former des propriétés de fixation qui sont configurées pour assurer sélectivement un support, et pour exposer au moins une portion du matériau métallique poreux.

14. Procédé selon la revendication 11, dans lequel la fixation du matériau métallique poreux comprend le soudage du matériau métallique poreux sur la première surface supérieure de substrat.

15. Procédé selon la revendication 11, dans lequel la fixation du matériau métallique poreux comprend un parmi le soudage par diffusion ou métallurgique du substrat au matériau métallique poreux.
